# EUROPEAN PATENT SPECIFICATION

(11) **EP 1 435 239 B1**
(45) Date of publication and mention of the grant of the patent: **03.05.2006**
(21) Application number: 02293275.0
(22) Date of filing: 31.12.2002
(51) Int. Cl.: A61K 31/4422, A61K 9/16, A61K 9/22, A61P 9/12

(54) **Stabilized and easily processable granule of amlodipine maleate**
Stabilisiertes und verarbeitungsleichtes Granulat von Amlodipin Maleat
Granule stable et facile à mettre en forme d'amlodipine maléate

(43) Date of publication of application: 07.07.2004
(73) Proprietor: Cimex Pharma AG, 4102 Benningen/Basel (CH)
(72) Inventor: Seth, Pawan, Irvine, CA 92612 (US)
(74) Representative: Hirsch & Associés

(56) References cited:
- EP-A- 1 266 654
- WO-A-02/053134

## Description

### BACKGROUND OF THE INVENTION

The invention relates to a pharmaceutical composition for administration by oral route containing Amlodipine maleate salt and to the process for preparing this composition.

Amlodipine and its salts are potent and long acting channel blockers useful as antihypertensive agents.

Amlodipine besylate marketed under the trade name Norvasc® is the most employed salt, due to its high chemical stability. Norvasc® is formulated for oral administration under the tablet form associated with various suitable excipients known by the person skilled in the art.

Amlodipine maleate is the most preferred salt for its toxicological properties presented in European patent EP 89167. However, this salt is also known to show less stability than Amlodipine besylate during the storage of the formulation in standard conditions of temperature and relative humidity, even over short periods, such as 6 months. Moreover US patent 4 879 303 by Davison et al. teaches that amlodipine maleate is one of the least stable salts. According to said patent, processability of the maleate salt is also much lower when compared to the besylate because of a high tendancy to stick to the tablet punches exhibited by the maleate salt

Hence, there is a need to obtain a composition of amlodipine maleate for which the processability as well as the chemical stability during storage, when tested in stability assays, is equivalent to that of the composition available on the market.

WO-A-97 12581 discloses pharmaceutical compositions designed for oral administration of acid-labile substances (benzimidazoles), which have excellent storage stability together with stability during their preparation process.

These compositions contain a core including the active principle and a binder deposited on a hydrosoluble inert support such as lactose associated with other suitable excipients, those formulations being e.g. granules.

WO-A-98 31360 discloses pharmaceutical compositions designed for oral administration comprising a hydrosoluble inert support such as lactose in association with a hydrophile polymer such as polyvinylpyrrolidone. In this case the composition which contains more than 20% by weight of polyvinylpyrrolidone shows an improved dissolution profile. Nothing is said about stability of the composition.

The use of polyvinylpyrrolidone is also known for manufacturing "solid dispersions" obtained in general by co-precipitation, co-fusion or liquid-phase mixing followed by drying. What we have here is fixation of the active ingredient in isolated microparticles on the polyvinylpyrrolidone, which avoids problems of poor wetting of the solid and re-agglomeration of the particles. The article "Stable Solid Dispersion System Against Humidity" by Kuchiki et al., Yakuzaigaku, 44 No. 1, 31-37 (1984) describes such a technique for preparing solid dispersions using polyvinylpyrrolidone. The amounts of PVP here are very high, and the ratio between the active ingredient and PVP are comprised between 1/1 and 1/20. In this case however there is no inert carrier.

WO02053134 describes stable compositions of amlodipine maleate, but fails to say that the use of between 2-30% by granule weight of PVP will confer stability to the compositon during storage.

### SUMMARY OF THE INVENTION.

Applicant has now found that the specific choice of polyvinylpyrrolidone(PVP) as a binder in compositions containing amlodipine maleate with an inert support provides an amlodipine composition with high stability during storage.

Tests with different binders known in the art such as hydroxypropylmethylcellulose, corn starch etc... lead to a degradation of amlodipine maleate during storage of the formulation, even over relatively short periods, such as 6 months at 40°C.

It is very surprising that only polyvinylpyrrolidone allows to reach a much higher stability with the composition of the invention as described below. The invention provides:
1. Granule comprising between 1 and 15% by granule weight of amlodipine maleate salt and between 2 and 30% by granule weight of polyvinylpyrrolidone and an inert carrier.
2. Granule according to item 1 wherein the inert carrier is hydrosoluble.
3. Granule according to item 2 comprising between 3 and 20% by granule weight of polyvinylpyrrolidone.
4. Granule according to items 1 to 3 comprising between 2 and 30 mg by weight of amlodipine maleate salt and between 4 and 100 mg by weight of polyvinylpyrrolidone.
5. Granule according to items 1 to 4 wherein the weight ratio amlodipine maleate salt:polyvinylpyrrolidone is between 10/1 and 1/50.
6. A tablet composition comprising granules according to items 1 to 5 which are compressed together.
7. A process for preparing a granule according to any one of items 1 to 5 comprising the steps of:
   a) preparing an amlodipine maleate salt suspension in a solution of polyvinylpyrrolidone;
   b) granulating the inert carrier with the suspension obtained from step (a);
   c) optionally, coating the granules thus obtained.
8. A process according to item 7 wherein the step b) is conducted in a fluidized bed.
9. A process according to item 7 or 8 for preparing a tablet according to item 6 comprising a step in which products obtained from step (b) or (c) are compressed.

### DETAILED DESCRIPTION.

The invention will apply to dry compositions for oral use such as granule comprising amlodipine maleate salt,
with polyvinylpyrrolidone and an inert carrier, preferably a hydrosoluble carrier. The granule will present a chemical stability at least equivalent to chemical stability of amlodipine besylate granule.

This improvement of the chemical stability of the composition of amlodipine maleate allows the product formulated according to the invention to retain its activity during all the period of the stability assays necessary for the determination of the expiry date of the drug (expiry date can be up to 3 or 5 years).

The polyvinylpyrrolidone (PVP) used in this invention is a synthetic water soluble homopolymer consisting of N-vinyl pyrrolidone and has, for example, a molecular weight comprised between 8,000 and 1,300,000, preferably for example between 30,000 and 300,000.

The granule according to the invention comprises, in general, an inert carrier from 10 to 90% by weight of the granule and preferably 60 to 90% by weight of the granule.
The active ingredient, the amlodipine maleate salt represents generally from 1 to 15% of the granule weight. The amount of polyvinylpyrolidone is generally comprised between 2 and 30% of the granule weight, preferably between 3 and 20% of the granule weight.

According to another embodiment the granule of the present invention comprises between 2 and 30 mg by weight of amlodipine maleate salt and between 4 and 100 mg by weight of polyvinylpyrolidone. The weight ratio amlodipine maleate salt:polyvinylpyrolidone can be comprised between 10/1 and 1/50.

In the framework of this invention, the expression "inert carrier" means any excipient, generally hydrophilic, pharmaceutically inert, crystalline or amorphous, in a particulate form, not leading to a chemical reaction under the operating conditions employed, and which is soluble in an aqueous medium, notably in a gastric acid medium, or sufficiently hydrophilic to be readily dispersible in such a medium. Examples of such excipients are derivatives of sugars, such as lactose, saccharose, or colloidal silica, or starch, hydrolyzed starch, (malto-dextrine), or cellulose, etc. Mixtures are also suitable. The individual particle size of the inert carrier can be, for example, between 50 and 500 micron.

The preferred carrier is hydrosoluble and the more particularly preferred carrier is lactose.

The compositions according to the invention can additionally contain any excipient conventionally used in the pharmaceutical and chemical fields which is compatible with the active ingredient, such as binders, fillers, pigments, disintegrating agents, lubricants, wetting agents, buffers, etc. These excipients able to be used in this invention are cited in "Handbook of pharmaceutical excipients" from Wade and Weller: The Pharmaceutical Press, London 1994, ISBN-91730-66-8.

The present invention provides also the pharmaceutical forms for oral use as tablets prepared from the above granule by compression.
It is also possible to provide, on the granules, an outer layer comprising additives, for the manufacture of tablets. In this embodiment, the outer layer comprises a disintegration agent and, for example, a lubricant; the thus covered and mixed granules can then be readily compressed and easily disintegrate in water. Examples of disintegrants are cross-linked polyvinylpyrrolidone, sodium croscarmellose, sodium carboxymethyl starch, modified starch or unmodified starch. Examples of lubricants are magnesium stearate, sodium stearyl fumarate, glycerol behenate and talc. Flow enhancing agents (such as colloidal silica) may also be used. The excipients can be any excipient traditionally used in the art. For more details about these excipients, one will refer to the disclosure in "Handbook of pharmaceutical excipients", American Pharmaceutical Association, 1994 ISBN 0 91730 66 8, by Wade A. and Weller P.

The granules will generally represent 50 to 90% of the final weight of the pharmaceutical tablet.

Another object of the invention is a process for preparing granule comprising the following steps:
a) preparing an amlodipine maleate salt suspension in a solution of polyvinylpyrolidone, for example in water or in an alcoholic solvent or a hydroalcoolic mixture.
b) granulating e.g. applying the suspension from step (a) to an inert carrier such as lactose by using classical granulation process such as wet granulation or oscillating mesh granulation, or by using fluidized bed granulation techniques;
   Applicant has found that the sticking tendency of amlodipine maleate reported in US patent 4 879 303 can be dramatically reduced by processing the granules in a fluidized bed equipment. Moreover, with this technique, the solvent is dried out during the granulation itself, allowing a larger amount of PVP to be used than in the classical granulation process. Therefore, according to a preferred embodiment the granulating step in the process for preparing granule is carried out in a fluidized bed.
c) Optionally, the granules thus obtained can be coated with classical excipients ;
   In order to prepare tablets the process can comprise a step in which products obtained from step (b) or (c) are compressed. This process is described in WO-A-98 31360.

### EXAMPLES

The following examples illustrate the invention without limiting it.

### Example 1.

The following formulations F1 a, b, c with PVP are prepared.

### Formulation 1a

| Compound F1a | Amount (mg) | %/ total |
|---|---|---|
| Amlodipine maleate | 12.8 | 6.6% |
| Povidone K30 | 30.00 | 15.5% |
| Lactose 60 mesh | 150.00 | 77.8% |
| (deionised water) | 120.00 | |
| Total dry weight | 192.8 | 100 |

### Formulation 1b

| Compound F1b | Amount (mg) | %/ total |
|---|---|---|
| Amlodipine maleate | 12.8 | 7.4% |
| Povidone K30 | 10.0 | 5.8% |
| Lactose 60 mesh | 150.00 | 86.8% |
| (deionised water) | 120.00 | |
| Total dry weight | 172.8 | 100.0% |

### Formulation 1c

| Compound F1c | Amount (mg) | %/ total |
|---|---|---|
| Amlodipine maleate | 12.8 | 7.6% |
| Povidone K30 | 5.0 | 3% |
| Lactose 60 mesh | 150.00 | 89.4% |
| (deionised water) | 120.00 | |
| Total dry weight | 167.8 | 100.0% |

The binder (Povidone) is dissolved in water, without preheating. Amlodipine maleate is then added to the solution. This suspension is sprayed onto lactose in the fluidized-bed apparatus Glatt GPCG-1 equipped with a top-spraying system (Top-Spray), using the following parameters of spraying :

| | |
|---|---|
| Input temperature | 55°C |
| Product temperature | 30°C |
| Flow-rate spraying | 11 g/min |
| Flow-rate air | 85 m3/h |
| Spray pressure | 2.6 10⁵ Pa. |

At the end of the spraying, the granules are dried directly in the fluidized-bed apparatus during 20 minutes at a temperature of 40°C.

### Example 2

The following formulation F2 (comparative formulation) is prepared as described in example 1.

| Compound F2 | Amount (mg) | %/ total |
|---|---|---|
| Amlodipine maleate | 12.8 | 7.4 |
| HPMC 3cPs | 9.00 | 5.2 |
| Lactose 60 mesh | 150.00 | 87.3 |
| (deionised water) | 120.00 | |
| Total dry weight | 171.8 | 100 |

### Example 3

The following formulation F3 (comparative formulation) is prepared as described in example 1.

| Compound F3 | Amount (mg) | %/ tot. Dry Wt. |
|---|---|---|
| Amlodipine maleate | 12.8 | 7.6 |
| corn starch | 5.00 | 2.9 |
| Lactose 60 mesh | 150.00 | 89 |
| (deionised water) | 120.00 | |
| Total dry weight | 167.8 | 100 |

In this case, the starch is dispersed without preheating in water and the suspension is heated during 20 minutes at 80°C to obtain a starch, then cooled down to room temperature.

### Example 4

### Preparation of the tablets.

The obtained granules are then sieved on a 1.2 mm mesh and mixed with the remaining excipients according to the following formulations :

| compound | Amount (mg) F1a | Amount (mg) F1b | Amount (mg) F1c | Amount (mg) F2 | Amount (mg) F3 |
|---|---|---|---|---|---|
| Granule | 192.8 | 172.8 | 167.8 | 171.8 | 167.8 |
| microcrystalline Cellulose | 40.00 | 40.00 | 40.00 | 40.00 | 40.00 |
| Crospovidone | 30.00 | 30.00 | 30.00 | 30.00 | 30.00 |
| sodium Stearylfumarate | 2.00 | 2.00 | 2.00 | 2.00 | 2.00 |
| TOTAL | 264.8 | 244.8 | 239.8 | 243.8 | 239.8 |

The mixing is performed in a mixer of the Turbula type (WA Bachhoffen, Swiss) at a speed of 25 rpm for 5 minutes.

The tablets are obtained from this mixture on a Fette press of type P2 equipped with concave dye of 9mm diameter and 9mm curve radius. The degree of compression is adjusted so as to reach a hardness of around 90N (Durometer of Schleuniger).

### Stability results

The tablets once made are packaged into polypropylene vials in accordance to the most recent Edition of the US Pharmacopoeia and placed in an incubator at 40°C and 75% of relative humidity during a 6 months period, then removed from the incubator and analysed according to the following procedure :

| | |
|---|---|
| Mobile phase | 15% acetonitrile/35% methanol/ 50% buffer (7ml of triethylamine + 1 litter of water, adjust the pH to 3.0 with orthophosphoric acid) |
| Column | nucleosil C18 5µ 150X4,6. mm |
| Detection : | 237nm |
| Flow-rate : | 1ml/min |
| Injected volume : | 20µl |
| Temperature : | 25°C |
| Retention time | 5 min |

The results obtained are the following (expressed as a percentage of the initial concentration of amlodipine maleate).

| | F1a | F1b | F1c | F2 | F3 |
|---|---|---|---|---|---|
| Concentration in % of the initial value | 99.6% | 99.1% | 98.3% | 92.6% | 94.1% |

It appears clearly from these results that the formulation according to the present invention, which contains polyvinypyrrolidone, is much more stable that those containing other binders.

## Claims

1. Granule comprising between 1 and 15% by granule weight of amlodipine maleate salt and between 2 and 30% by granule weight of polyvinylpyrrolidone and an inert carrier.

2. Granule according to claim 1 wherein the inert carrier is hydrosoluble.

3. Granule according to claim 2 comprising between 3 and 20% by granule weight of polyvinylpyrrolidone.

4. Granule according to claims 1 to 3 comprising between 2 and 30 mg by weight of amlodipine maleate salt and between 4 and 100 mg by weight of polyvinylpyrrolidone.

5. Granule according to claims 1 to 4 wherein the weight ratio amlodipine maleate salt:polyvinylpyrrolidone is between 10/1 and 1/50.

6. A tablet composition comprising granules according to claims 1 to 5 which are compressed together.

7. A process for preparing a granule according to any one of claims 1 to 5 comprising the steps of
a) preparing an amlodipine maleate salt suspension in a solution of polyvinylpyrrolidone;
b) granulating the inert carrier with the suspension obtained from step (a);
c) optionally, coating the granules thus obtained.

8. A process according to claim 7 wherein the step b) is carried out in a fluidized bed.

9. A process according to claim 7 or 8 for preparing a tablet according to claim 7 comprising a step in which products obtained from step (b) or (c) are compressed.

## Patentansprüche

1. Granulat bzw. Granalien, umfassend zwischen 1 und 15%, bezogen auf das Granulatgewicht, Amlodipin-Maleatsalz und zwischen 2 und 30%, bezogen auf das Granulatgewicht, Polyvinylpyrrolidon und einen inerten Träger.

2. Granulat gemäß Anspruch 1, wobei der inerte Träger wasserlöslich ist.

3. Granulat gemäß Anspruch 2, umfassend zwischen 3 und 20%, bezogen auf das Granulatgewicht, Polyvinylpyrrolidon.

4. Granulat gemäß den Ansprüchen 1 bis 3, umfassend zwischen 2 und 30 mg, bezogen auf das Gewicht, Amlodipin-Maleatsalz und zwischen 4 und 100 mg, bezogen auf das Gewicht, Polyvinylpyrrolidon.

5. Granulat gemäß den Ansprüchen 1 bis 4, wobei das Gewichtsverhältnis von Amlodipin-Maleatsalz:Polyvinylpyrrolidon zwischen 10/1 und 1/50 liegt.

6. Tablettenzusammensetzung, umfassend Granalien gemäß den Ansprüchen 1 bis 5, welche miteinander verpreßt sind.

7. Verfahren zur Herstellung eines Granulats gemäß einem der Ansprüche 1 bis 5, umfassend die Schritte
a) des Herstellens einer Amlodipin-Maleatsalz-Suspension in einer Lösung von Polyvinylpyrrolidon;
b) des Granulierens des inerten Trägers mit der in Schritt (a) erhaltenen Suspension;
c) gegebenenfalls des Beschichtens des derart erhaltenen Granulats.

8. Verfahren gemäß Anspruch 7, wobei der Schritt b) in einem fluidisierten Bett durchgeführt wird.

9. Verfahren gemäß Anspruch 7 oder 8 zur Herstellung einer Tablette gemäß Anspruch 7, umfassend einen Schritt, worin die in Schritt (b) oder (c) erhaltenen Produkte verpreßt werden.

## Revendications

1. Granulé comprenant entre 1% et 15% en poids du granulé de maléate d'amlodipine et entre 2% et 30% en poids du granulé de polyvinylpyrrolidone et un véhicule inerte.

2. Granulé selon la revendication 1, dans lequel le véhicule inerte est hydrosoluble.

3. Granulé selon la revendication 2 comprenant entre 3% et 20% en poids du granulé de polyvinylpyrrolidone.

4. Granulé selon les revendications 1 à 3 comprenant entre 2 et 30 mg en poids de maléate d'amlodipine et entre 4 et 100 mg en poids de polyvinylpyrrolidone.

5. Granulé selon les revendications 1 à 4, dans lequel le rapport pondéral maléate d'amlodipine:polyvinylpyrrolidone est compris entre 10:1 et 1:50.

6. Composition pour comprimé comprenant des granulés selon les revendications 1 à 5 compressés ensemble.

7. Procédé de préparation d'un granulé selon l'une quelconque des revendications 1 à 5 comprenant les étapes consistant à :
a) préparer une suspension de maléate d'amlodipine dans une solution de polyvinylpyrrolidone ;
b) granuler le véhicule inerte avec la suspension obtenue à l'étape (a) ;
c) éventuellement, enrober les granulés ainsi obtenus.

8. Procédé selon la revendication 7, dans lequel l'étape (b) est réalisée dans un lit fluidisé.

9. Procédé selon la revendication 7 ou 8 de préparation d'un comprimé selon la revendication 7 comprenant une étape dans laquelle les produits obtenus à l'étape (b) ou (c) sont compressés.
